Europäisches Patentamt

(19) European Patent Office

Office européen des brevets

(11) Publication number: **0 055 609**

**A1**

(12) **EUROPEAN PATENT APPLICATION**

(21) Application number: **81306107.4**

(22) Date of filing: **23.12.81**

(51) Int. Cl.³: **A 61 K 31/635**
**A 61 K 9/02, A 61 K 31/685**
**//(A61K31/685, 31/635, 31/505)**

(30) Priority: **26.12.80 JP 185780/80**

(43) Date of publication of application:
**07.07.82 Bulletin 82/27**

(84) Designated Contracting States:
**CH DE FR IT LI SE**

(71) Applicant: **Shionogi & Co., Ltd.**
**12, 3-chome Dosho-machi Higashi-ku**
**Osaka(JP)**

(72) Inventor: **Takagishi, Yasushi**
**5-22, Horikiri-cho**
**Nishinomya Hyogo(JP)**

(72) Inventor: **Ohsuga, Kiichiro**
**376-22, Kawara-cho**
**Kusatsu Shiga(JP)**

(72) Inventor: **Ohama, Sadao**
**448-3, Nagata Onoe-cho**
**Kakogawa Hyogo(JP)**

(74) Representative: **Bizley, Richard Edward et al,**
**BOULT, WADE & TENNANT 27 Furnival street**
**London EC4A 1PQ(GB)**

(54) Composition for coelomic administration, its preparation, and ingredients for use therein.

(57) A pharmaceutical or veterinary composition for coelomic administration comprising sulfamethoxazole and trimethoprim with a base material(s), having a phospholipid included therein so that the tendency of solidification at the step of mixing of the said components in the manufacture of the said composition is prevented whilst solving the problem of delayed disintegration. When trimethoprim is used in the form of its salt or together with an organic acid in the manufacture of the said composition, the absorption rate of trimethoprim can be improved.

EP 0 055 609 A1

COMPOSITION FOR COELOMIC ADMINISTRATION, ITS

PREPARATION, AND INGREDIENTS FOR USE THEREIN

The present invention relates to a pharmaceutical composition for coelomic administration and its preparation. More particularly, it relates to a pharmaceutical composition comprising sulfamethoxazole and trimethoprim, which is suitable for administration through a body cavity, and a process for preparing the same.

Sulfamethoxazole and trimethoprim are known as antimicrobial agents. It is also well known that the combined use of them within a certain proportion ranges produces a synergistic effect in antimicrobial activity (U.S. patent 3,341,541). Thus, the combination of sulfamethoxazole and trimethoprim in a weight proportion of 5 : 1 is normally used, although a higher sulfamethoxazole proportion is practically usable. In fact, tablets comprising 400 mg of sulfamethoxazole and 80 mg of trimethoprim per each tablet are on the clinical use with an ordinary dose of two tablets twice a day. This corresponds to the administration of 800 mg of sulfamethoxazole and 160 mg of trimethoprim each time.

Since the said tablets contain a relatively great amount of the active ingredients, their size are obliged to be large. This causes difficulty on the oral administration. Further, they sometimes cause a damage to a principal absorption organs such as stomach or duodenum.

In order to avoid the above difficulty, it was frequently tried to administer the active ingredients through a body cavity such as rectum.  From the practical viewpoint, however, the following drawbacks are seen on the realization of such coelomic administration:

(1)  In the manufacture of a preparation suitable for coelomic administration, the viscosity of a mixture of the active ingredients and a base material(s) at the mixing step is abnormally elevated to solidify, and the resulting mixture is hardly molded into a desired shape;

(2)  The preparation as manufactured changes remarkably in various physical properties such as disintegrating property, dissolving property and melting property, and in particular, the disintegration is unfavorably delayed.

(3)  Even immediately after the manufacture, the preparation can hardly produce desired blood levels of the active ingredients.

Various proposals have been made to overcome the above drawbacks.  For instance, German Offenlegungsschrift No. 2,415,660 proposes a suppository preparation comprising a portion containing sulfamethoxazole alone and a portion containing trimethoprim alone, optionally with any separation layer between them.  This proposal overcomes the difficulty on the manufacture to a certain extent but is disadvantageous in requiring two or three stages for molding.  Further, for instance, Japanese Patent Publication (unexamined) No. 102,517/80 proposes a preparation for coelomic administration using a complex of sulfamethoxazole with trimethoprim as disclosed in Japanese patent 805,379 (Japanese Patent Publication (examined) No. 20125/75).

As a result of the subsequent study for overcoming the solidification at the step for mixing sulfamethoxazole and trimethoprim as the active ingredients with a base material(s), it has now been found that the presence of a small amount of a phospholipid in the mixing system is effective in prevention of such solidification. Advantageously, the phospholipid can solve simultaneously the problem of delay in disintegration. It has also been found that the use of trimethoprim in its salt or the incorporation of an organic acid into the mixture can promote the absorption of trimethoprim, its own absorption rate being somewhat smaller than that of sulfamethoxazole. The present invention is based on the above findings.

Accordingly, a basic object of the present invention is to provide a pharmaceutical or veterinary composition comprising sulfamethoxazole and trimethoprim, which is suitable for coelomic administration. Another object of this invention is to provide a process for preparing a pharmaceutical or veterinary composition comprising sulfamethoxazole and trimethoprim, which can be prepared while preventing the solidification at the step for mixing of the active ingredients with a base material(s). A further object of the invention is to provide a pharmaceutical or veterinary composition comprising sulfamethoxazole and trimethoprim, wherein the absorption of trimethoprim is improved without affording any unfavorable influence on the absorption of sulfamethoxazole.

According to the present invention, there is provided a pharmaceutical or veterinary composition for coelomic administration comprising sulfamethoxazole and trimethoprim with a base material(s), and having a phospholipid included therein so that the tendency of solidification at the step for mixing the said components in the manufacture of the said composition is prevented, whereby the said composition can be molded into any desired shape suitable for coelomic administration without any difficulty. Advantageously, the inclusion of the phospholipid can solve at the same time the problem of the delayed disintegration.

The term "pharmaceutical or veterinary composition for coelomic administration" is intended to mean any pharmaceutical composition which is formulated in any preparation suitable for administration through a body cavity. It is usually manufactured as a suppository preparation, which maintains a solid state at room temperature and is readily melted in a body cavity. It may be also manufactured in the form of ointment of liquid, which can be filled in a soft capsule for administration.

The term "sulfamethoxazole" as one of the active ingredients in the composition of the invention covers not only sulfamethoxazole itself but also its derivatives which are considered to exert an antimicrobial activity substantially in the form of sulfamethoxazole in a living body. Examples of such derivatives
are acetylsulfamethoxazole, sulfamethoxazole sodium, etc.

Likewise, the term "trimethoprim" as the other
active ingredient covers not only trimethoprim itself but
also its derivatives which are considered to exert an
antimicrobial activity substantially in the form of trimethoprim in a living body. Examples of such derivatives
are organic and inorganic salts of trimethoprim such as
hydrochloride, sulfate, citrate, lactate, tartrate, malate
and maleate. In this connection, it should be noted that
the incorporation of trimethoprim in its salt form into the
composition of the invention can promote the absorption of
trimethoprim. Alternatively, trimethoprim or its salt may
be incorporated into the composition with any organic acid
(e.g. lactic acid, citric acid, tartaric acid, maleic acid,
malic acid, glutamic acid, aspartic acid) so that the
absorption rate of trimethoprim can be increased. When the
organic acid is incorporated, its amount may be usually from
about 0.1 to 4 % by weight based on the weight of the
composition.

In the composition of the invention, the weight
proportion of sulfamethoxazole and trimethoprim as the
active ingredients may be usually from about 1 : 1 to 10 :
1. The composition of the invention may be administered in
such an amount as providing from about 1 to 50 mg of sulfamethoxazole and from about 1 to 5 mg of trimethoprim per kg of the body weight for a single dose. The interval of administration is usually from about 12 to 18 hours.

As the phospholipid, there may be used any phospholipid obtained from plant seeds such as soybean, eggs, livers of animals such as cows and pigs, etc. Synthetic ones are also usable. Specific examples are phosphatidylcholine, phosphatidylethanolamine, phosphatidylserine, phosphatidylinositol, sphingomyelins, dipalmitoyllecithin, etc. These may be employed alone or in combination. The amount of the phospholipid to be used may be from about 0.5 to 5 % by weight based on the weight of the composition. When the amount is less than the said lower limit, the desired solidification preventing effect is not exerted. When the amount is more than the said upper limit, the viscosity of the composition is lowered. Thus, the composition becomes sticky so that its molding into an appropriate shape is quite difficult. In addition, the quality of the composition is much deteriorated.

As the base material(s), there may be used any physiologically accepable carrier or diluent conventionally employed for preparation of ointments or suppositories. Examples of the base material(s) are oils and fats (e.g. peanut oil, plam oil, olive oil, soybean oil, sesame oil, cotton seed oil, castor oil, linseed oil, rice bran oil, rape oil, corn oil, cacao butter, beef tallow, lanolin), their hydrogenated products, their modified products with

- 7 -

0055609

exchange of fatty acids or acetylation, their glycerides, etc. There are also usable water-miscible base materials such as polyethylene glycol, propylene glycol, glycero-gelatin, methylcellulose and carboxymethylcellulose.

In order to accelerate the absorption of the active ingredients, any surfactant which exerts only a mild action to mucous membranes may be incorporated in the composition of the invention. The surfactant may be any of nonionic, cationic, anionic and amphoionic types. Specific examples are polyoxyethylene sorbitan fatty acid esters, polyoxyethylene higher alcohol esters, polyoxyethylene sorbitol fatty acid esters, polyoxyethylene fatty acid esters, polyoxyethylene alkylamines, polyoxyethylene alkylamides, polyether alcohol sulfates, alkyl sulfates, etc.

When desired, the composition of the invention may further include any other additive such as emulsifiers, stabilizers, preservatives, coloring agents, etc.

As to the order of incorporation of sulfamethoxazole, trimethoprim and the phospholipid with or without an organic acid into a base material(s), there is no particular limitation. Thus, they may be mixed together in any optional order.

Practical and presently preferred embodiments of the invention are illustratively shown in the following Examples wherein part(s) are by weight.

Example 1

(1)   Preparation of suppositories:-

A base material "Witepsol" (trademark; manufactured by Dynamit Nobel A.G.; a mixture of mono-, di- and tri-glycerides of $C_{12}$-$C_{18}$ saturated fatty acids) was melted at 50°C and admixed with a phospholipid (soya lecithin). After sufficient agitation, sulfamethoxazole and trimethoprim (or its lactate) were added thereto, and the resultant mixture was agitated well to make a uniform dispersion.   Every two grams of the resulting mixture were filled in molds and, after cooling, taken out therefrom to obtain suppositories.

In the above preparation, the components were used in the amounts as shown in Table 1.

Table 1

(Part(s))

| Composition Component | A | B | C (Control) |
|---|---|---|---|
| Sulfmethoxazole | 20 | 20 | 20 |
| Trimethoprim | 4 | - | 4 |
| Trimethoprim lactate | - | 5.2[*] | - |
| Phospholipid (soya lecithin) | 2 | 2 | - |
| Base material ("Witepsol") | 74 | 72.8 | 76 |
| Total | 100 | 100 | 100 |

Note:   *)   corresponding to 4 parts of trimethoprim.

(2)   Disintegration test:-

By the use of a penetrometer (manufactured by Erweka A.G.), the disintegration time of the suppository prepared above in physiologically saline solution at 37°C was measured. The results are shown in Table 2.

Table 2[*)]

(Minute(s))

| Storage<br><br>Composi-<br>tion | Immediately<br>after manu-<br>facture | After storage<br>at cold place<br>for 3 months | After storage<br>at room temper-<br>ature for 3<br>months |
|---|---|---|---|
| A | 7<br>(5 - 8) | 9<br>(7 - 10) | 13<br>(10 - 14) |
| B | 9<br>(7 - 10) | 10<br>(9 - 11) | 12<br>(11 - 13) |
| C | 18<br>(16 - 20) | 35<br>(25 - 40) | Not broken even<br>after 60 minutes |

Note: [*)] The value indicates an average of three measurements. The range of the values as found is indicated in parenthesis.

(3) Rectal absorption:-

The suppository (2 g) immediately after the preparation was administered to each Viegel dog (average body weight, 10 kg), fasted for about 17 hours and defeced immediately before the administration, through the anus to a depth of about 4 cm in the rectum. The blood was taken from the vein at the forepaw and centrifuged. The concentrations of sulfamethoxazole and trimethoprim in the obtained plasma were measured respectively by high speed liquid chromatography and by gas chromatography. The results are shown in Table 3 wherein the numerals are in average for 6 animals.

Table 3

(μg/ml)

| Composition | Active ingredients | Time (hr(s)) 0.5 | 1 | 2 | 3 | 5 | 7 | 9 | 12 | 24 |
|---|---|---|---|---|---|---|---|---|---|---|
| A | Sulfa-methoxazole | 20.8 | 53.7 | 69.9 | 68.5 | 64.1 | 60.0 | 53.2 | 47.7 | 29.0 |
| A | Trimethoprim | 0.24 | 0.38 | 0.70 | 0.96 | 1.06 | 0.98 | 0.96 | 0.84 | 0.39 |
| B | Sulfa-methoxazole | 16.8 | 46.4 | 61.2 | 72.5 | 70.0 | 62.3 | 56.2 | 49.0 | 28.3 |
| B | Trimethoprim | 0.52 | 0.80 | 1.05 | 1.26 | 1.18 | 1.09 | 0.91 | 0.75 | 0.31 |
| C (Control) | Sulfa-methoxazole | 4.3 | 16.8 | 28.3 | 40.2 | 43.0 | 39.7 | 36.8 | 34.5 | 2.07 |
| C (Control) | Trimethoprim | 0.04 | 0.21 | 0.36 | 0.45 | 0.52 | 0.55 | 0.51 | 0.45 | 0.20 |

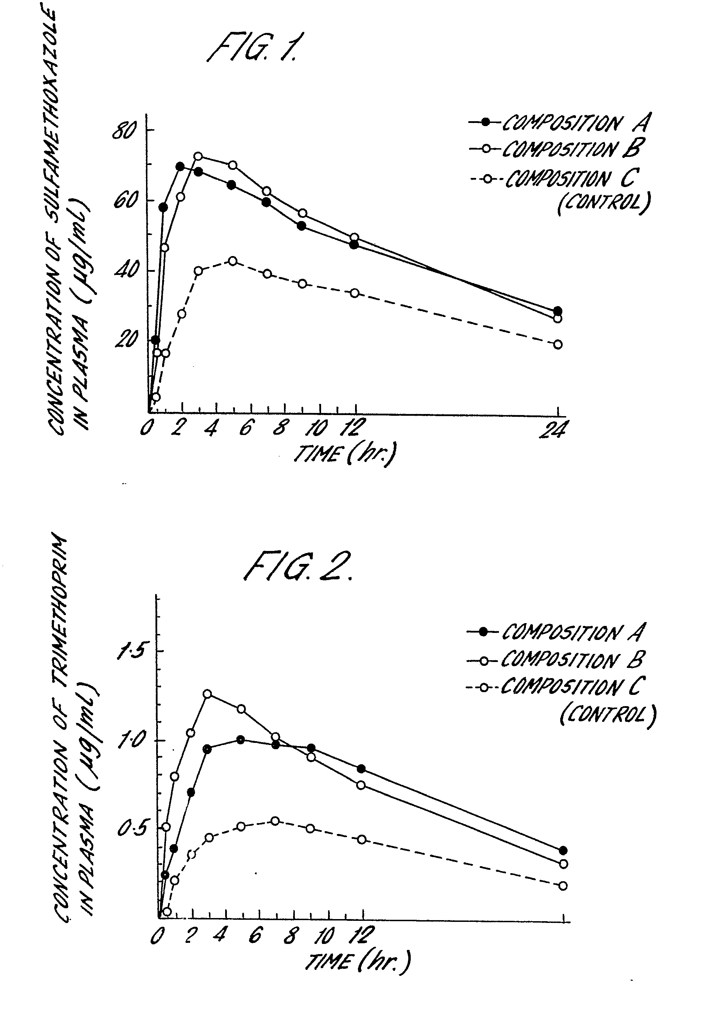

The numerals in Table 3 were plotted on graphs to give Figures 1 and 2 of the accompanying drawings. Fig. 1 indicates the relationship of the sulfamethoxazole concentration in plasma ($\mu$g/ml) on the ordinate with the time elapsed (hour(s)) on the abscissa, and Fig. 2 indicates the relationship of the trimethoprim in plasma ($\mu$g/ml) on the ordinate with the time elapsed (hour(s)) on the abscissa.

Example 2

"Witepsol" (71.4 g) was melted at 50°C and admixed with a phospholipid (soya lecithin) (2 g), followed by agitation to make a uniform mixture. Into the mixture, sulfamethoxazole (20 g), trimethoprim (4 g) and citric acid (2.6 g) were dispersed uniformly. Every two grams of the resultant mixture were filled in molds and, after cooling, taken out therefrom to obtain suppositories (Composition D).

In the same manner as in Example 1, the disintegration test was carried out. The results are shown in Table 4.

Table 4

(Minute(s))

| Storage | Immediately after manufacture | After storage at cold place for 3 months | After storage at room temperature for 3 months |
|---------|-------------------------------|-------------------------------------------|------------------------------------------------|
| Composition D | 9 | 10 | 12 |

Example 3

In the same manner as in Example 1, the following suppositories were prepared:

Composition E:-

| | | |
|---|---|---|
| Sulfamethoxazole | 20 | grams |
| Trimethoprim | 8 | |
| Tartaric acid | 4 | |
| Phospholipid (york lecithin) | 2 | |
| "Witepsol" | 66 | |
| Total | 100 | grams |

Composition F:-

| | | |
|---|---|---|
| Sulfamethoxazole | 20 | grams |
| Trimethoprim lactate | 26 | (corresponding to 20 grams of trimethoprim) |
| Glutamic acid | 6 | |
| Phospholipid (bovine lecithin) | 8 | |
| "Witepsol" | 140 | |
| Total | 200 | grams |

In the same manner as in Example 1, the suppositories were subjected to the disintegration test. The similar results were obtained.

Example 4

To sesame oil (68 g) warmed at 40°C, a phospholipid (phosphatidylethanolamine) (4 g) was added, followed by sufficient agitation. To the resultant mixture, sulfamethoxazole (20 g), trimethoprim (4 g) and glutamic acid (4 g) were added to make a uniform dispersion. Every one gram of the resulting mixture was filled in gelatin soft capsules (Composition G).

CLAIMS:

1. A pharmaceutical or veterinary composition suitable for coelomic administration comprising sulfamethoxazole, trimethoprim, one or more base materials, and a phospholipid.

2. A composition as claimed in claim 1, wherein the phospholipid is included in an amount of from about 0.5 to 5% by weight based upon the weight of the composition.

3. A composition as claimed in claim 1 or claim 2, wherein trimethoprim is included in the form of a salt thereof.

4. A composition as claimed in any one of claims 1 to 3, wherein an organic acid is included therein.

5. A composition as claimed in any one of claims 1 to 4, wherein the weight ratio of sulfamethoxazole to trimethoprim is from about 1:1 to 10:1.

6. A process for preparing a pharmaceutical or veterinary composition suitable for coelomic administration, which process comprises mixing sulfamethoxazole and trimethoprim with one or more base materials in the presence of a phospholipid.

7. A process as claimed in claim 6, wherein the phospholipid is present in an amount of from about 0.5 to 5% by weight based upon the weight of the composition.

8. A process as claimed in claim 6 or claim 7, wherein the mixing is effected in a molten or liquid state.

9.    A process as claimed in claim 8, wherein after the mixing a mold is filled by the resulting mixture and, after cooling, the resulting shaped product is removed from the mold to obtain a suppository.

10.   Sulfamethoxazole, trimethoprim, and/or a phospholipid for use, in the form of a coelomically administered composition comprising all three of said substances, in producing an antimicrobial effect in a human being or other animal.

0055609

1/1

FIG. 1.

COMPOSITION A
COMPOSITION B
COMPOSITION C (CONTROL)

FIG. 2.

COMPOSITION A
COMPOSITION B
COMPOSITION C (CONTROL)

0055609

Application number

EP 81 30 6107

**European Patent Office**

**EUROPEAN SEARCH REPORT**

## DOCUMENTS CONSIDERED TO BE RELEVANT

| Category | Citation of document with indication, where appropriate, of relevant passages | Relevant to claim |
|---|---|---|
| A | GB - A - 2 041 749 (SHIONOGI & CO. LTD.) <br><br> * page 5, line 22 - page 6, line 44; claims 1-26 * | 1-10 |
| D,A | & JP - A - 55 10 2517 | |
| | --- | |
| A | DE - C - 667 500 (CHEM. PHARM. AG. BAD HOMBURG) <br><br> * page 3, lines 19-36; claims 1-2 * | 1-10 |
| | --- | |
| P,A | DE - A - 2 951 142 (A. NATTERMANN & CIE) <br><br> * page 1, claims 1-5 * | 1-10 |
| | --- | |
| A | ROTE LISTE, 1979 <br> Editio Cantor <br> AULENDORF/WÜRTT. <br> no. 77 029B  CO-TRIMOXAZOL <br> no. 77 030B  CO-TRIM-TABLINEN <br> no. 77 031B  DRYLIN <br> no. 77 032B  DURATRIMET <br> no. 77 033B  EUSAPRIM <br> no. 77 035B  KEPINOL <br> no. 77 037B  MICROTRIM <br> no. 77 038B  OMSAT <br> no. 77 039B  SIGAPRIM <br> no. 77 040B  SULFACET <br> no. 77 041B  SULFOTRIMIN <br> no. 77 043B  TMS 480 <br> no. 77 044B  TRIGONYL <br> no. 77 045B and 77 046B TRIMETHO-PRIM COMP. RATIOPHARM <br> no. 77 047B  TRIEMETHOPRIM COMP. STADA | 1-10 |
| | --- | |
| D,A | DE - A - 2 415 660 (CHIESI P.) | 1-10 <br> ./. |

### CLASSIFICATION OF THE APPLICATION (Int. Cl. 3)

A 61 K 31/635
        9/02
       31/685
// (A 61 K 31/685
       31/635
       31/505)

### TECHNICAL FIELDS SEARCHED (Int.Cl. 3)

A 61 K
C 07 D

### CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| | | | |
|---|---|---|---|
| X | The present search report has been drawn up for all claims | | |

| Place of search | Date of completion of the search | Examiner |
|---|---|---|
| The Hague | 23-03-1982 | BRINKMANN |

EPO Form 1503.1  06.78

**European Patent Office**

**EUROPEAN SEARCH REPORT**

Application number

EP 81 30 6107

-2-

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| D,A | <u>US - A - 3 341 541</u> (MAX HOFFER) | 1-10 | |

TECHNICAL FIELDS SEARCHED (Int. Cl.³)

EPO Form 1503.2   06.78